# FASCICULE DE BREVET EUROPEEN

(11) **EP 2 848 264 B1**
(45) Date de publication et mention de la délivrance du brevet: **23.12.2020**
(21) Numéro de dépôt: 14182959.8
(22) Date de dépôt: 29.08.2014
(51) Int. Cl.: A61L 2/10, C02F 1/32

(54) **Dispositif de stérilisation de liquide par rayonnement ultraviolet**
Vorrichtung zum Sterilisieren von Flüssigkeiten durch UV-Strahlung
Device for sterilising liquid by ultraviolet radiation

(30) Priorité: 11.09.2013 FR 1358742
(43) Date de publication de la demande: 18.03.2015
(73) Titulaire: Trefle Groupe, 91031 Evry (FR)
(72) Inventeur: Liccioni, Thomas, 92120 MONTROUGE (FR); Liccioni, Robert, 77150 LESIGNY (FR)
(74) Mandataire: Santarelli

(56) Documents cités:
- EP-A2- 0 430 581
- WO-A1-2007/035114
- FR-A1- 2 927 324
- US-A1- 2003 052 278

## Description

La présente demande concerne les dispositifs de traitement de liquide, comme par exemple l'eau.

Plus précisément, la présente demande concerne les dispositifs de traitement de liquide par rayonnement, comme par exemple les rayonnements ultraviolets.

De tels dispositifs sont notamment destinés à être disposés dans un appareil de distribution de boisson, comme par exemple une fontaine à eau, et en particulier près d'un point de distribution de la boisson.

On connait des dispositifs de traitement de l'eau comportant un système stérilisant par exemple par rayonnements ultraviolets. Cependant, ces dispositifs peuvent présenter une efficacité limitée au point de distribution, car bien souvent, l'extrémité du dispositif peut laisser une possibilité de rétro-contamination entre le point de distribution et le système stérilisant. L'art antérieur connaît également, par les brevets FR 2 927 324 et US 2003/0052278 A1, des dispositifs de traitement de liquide par rayonnements ultraviolets.

La présente invention vise à améliorer les dispositifs existants et mener en outre à d'autres avantages.

A cet effet, est proposé selon un premier aspect, un dispositif de désinfection d'un liquide par rayonnement ultraviolet comportant :
- un tronçon de conduit cylindrique (50) configuré pour que du liquide s'y écoule,
- au moins une source de rayonnement ultraviolet primaire disposée en périphérie extérieure du tronçon de conduit cylindrique, la source de rayonnement ultraviolet primaire comportant une lampe qui comporte deux cylindres coaxiaux, dont un cylindre externe et un cylindre interne formant le tronçon de conduit cylindrique, qui sont en un matériau diélectrique propre à transmettre des rayons ultraviolets et qui sont reliés par leurs extrémités de sorte à définir entre eux une chambre hermétique contenant un gaz, la lampe comportant en outre une première électrode et une seconde électrode, la première et la seconde électrode étant reliées à une source de courant alternatif, la lampe étant configurée pour générer un rayonnement ultraviolet entre les deux cylindres coaxiaux, le tronçon de conduit cylindrique et l'au moins une source de rayonnement ultraviolet étant configurés pour que l'au moins une source de rayonnement ultraviolet irradie le tronçon de conduit cylindrique et le liquide s'écoulant dans le tronçon de conduit cylindrique, depuis l'extérieur du tronçon de conduit cylindrique, et
- au moins un tube, formé en un matériau diélectrique propre à transmettre des rayons ultraviolets, qui prolonge le tronçon de conduit cylindrique en s'étendant depuis au moins une sortie du tronçon de conduit cylindrique jusqu'à au moins un point de distribution du dispositif, le tube étant configuré pour guider le rayonnement ultraviolet en provenance du tronçon de conduit cylindrique jusqu'à l'au moins un point de distribution et irradier le liquide s'y écoulant.

Le dispositif est un dispositif de désinfection fonctionnant avec une lampe à rayons ultraviolets à deux cylindres coaxiaux où l'eau qui le traverse est irradiée au centre de la lampe pour détruire les virus et bactéries. La constitution et le fonctionnement d'une telle lampe sont par exemple détaillés dans le document US 6 398 970.

Une telle source de rayonnement ultraviolet primaire est particulièrement compacte. Elle permet en outre un allumage du dispositif seulement lorsqu'une désinfection et nécessaire et permet ainsi d'éviter que le dispositif reste allumé en permanence, ce qui induit une économie d'énergie. En outre, sa configuration avec le liquide s'écoulant à l'intérieur du cylindre interne autour duquel est directement généré le rayonnement ultraviolet permet de plus faibles pertes entre le rayonnement généré émis et celui reçu par le fluide, notamment du fait que le nombre d'interfaces est réduit. Le dispositif selon l'invention permet donc d'avoir un dispositif compact, en minimisant les pertes de débit tout en ayant une exposition de l'écoulement au rayonnement maximisée du fait que l'écoulement est entouré par le rayonnement. Il y a ainsi un bon compromis entre la conservation du débit de l'écoulement et le temps d'exposition au rayonnement.

Selon un exemple de réalisation, le gaz remplissant la chambre hermétique comporte du xénon. Un tel gaz est par exemple un mélange à base de xénon ou du xénon sans oxygène.

Selon un autre exemple de réalisation, une partie de paroi interne de la chambre est revêtue d'un revêtement en un matériau luminescent émettant dans le domaine des ultraviolets, et de préférence dans le domaine des UV-C. Un tel revêtement contient par exemple du phosphore propre à émettre des rayons UV-C. Le revêtement peut optionnellement comprendre un liant, organique ou inorganique. Il comprend par exemple un matériau inorganique contenant de l'oxygène, tels que des oxydes, des sulfates, des phosphates aluminates, des borates ou des silicates. Et il comprend par exemple un activateur, tel qu'un ion métallique choisi dans le groupe formé par Pr³⁺, Bi³⁺ et Pb²⁺. Optionnellement, CaSO₄:Pb, SrSO₄:Pb, MgSO₄:Pb, (Ca,Mg)SO₄:Pb (Ca,Mg,Sr) SO₄:Pb, (Ca,Sr)SO₄:Pb, CaLi₂SiO₄:Pb, SrSiO₃:Pb, (Ca,Sr,Ba)SiO₃:Pb, Ba(Y,Gd,Lu)B₉O₁₆:Bi, YF₃Bi, YOF:Bi, (Gd,Y)OF:Bi,Pr, Y₃Al₅O₁₂:Bi, (Gd,Y)₃Al₅O₁₂:Bi, (Gd,Y)₃ (Al,Ga)₅O₁₂:Bi, (Ca,Y,Lu)PO₄:Pr, (Lu,Y)BO₃:Pr ou ScBO₃:PR peuvent convenablement être utilisé comme une matière luminescente, comme phosphores. En particulier, des phosphores préférés sont ceux comprenant du praséodyme, comme LaPO₄:Pr, LaB₃O₆:Pr, LaBo₃:Pr, YBO₃:Pr, YPO₄:Pr et Y₂SiO₅:Pr. D'autres phosphores préférés sont des phosphores comprenant du Bi, comme YPO₄: Bi et LuPO₄:Bi.

Ainsi, la source de rayons ultraviolet (UV) émet des rayons ultraviolet qui sont de préférence des rayons UV-C, c'est-à-dire par des rayons ayant généralement une longueur d'onde comprise entre environ 100 nm et 400 nm, et plus particulièrement une longueur d'onde comprise entre environ 153 nm et 280 nm pour les UV-C, et de préférence une longueur d'onde comprise entre environ 240 nm et 280 nm pour assurer une meilleure capacité de décontamination.

Le conduit du dispositif est par exemple formé par au moins le tube et le tronçon de conduit cylindrique.

On entend ici par « tronçon de conduit cylindrique » que le tronçon de conduit a globalement une section constante, de préférence circulaire, le long d'une ligne directrice droite. La source de rayonnement primaire est disposée à l'extérieur du tronçon de sorte à irradier l'ensemble du tronçon ainsi que le fluide s'écoulant à l'intérieur du tronçon.

De préférence, le tronçon de conduit cylindrique a une section circulaire, ce qui permet de minimiser des turbulences dans l'écoulement tout en maximisant son exposition au rayonnement. Un tel tronçon de conduit est en outre plus facilement réalisable.

De manière générale, les deux cylindres coaxiaux de la lampe ont une section circulaire pour faciliter la réalisation de la lampe tout en minimisant les turbulences possible dans l'écoulement de fluide.

La présence du tube en sortie du tronçon de tube permet ainsi de stériliser le liquide et le conduit jusqu'au point de distribution. Autrement dit, le tube permet ainsi de protéger le liquide en sortie du tronçon de conduit cylindrique jusqu'au point de distribution dans un dispositif de distribution d'eau équipé d'un stérilisateur. Il est ainsi possible de limiter des rétro-contaminations ou une accumulation de bactéries au point de distribution de manière simple et efficace puisque la stérilisation est principalement induite par la source de rayonnement primaire.

Le dispositif selon l'invention peut comprendre en outre tout ou partie des caractéristiques suivantes pouvant être combinées.

De manière générale, au moins une entrée, ou chaque entrée, du tronçon de conduit cylindrique est reliée à un conduit d'arrivée d'eau par un premier ensemble d'éléments de raccord. Le premier ensemble d'élément de raccord comprend au moins une pièce permettant de connecter et déconnecter à souhait le dispositif, ce qui permet d'avoir un dispositif facilement amovible dans tout type d'appareil de distribution de boisson. Ainsi, s'il faut changer la lampe par exemple, le démontage est alors très aisé.

L'au moins un point de distribution forme par exemple au moins un point d'extrémité du dispositif, c'est-à-dire que le point de distribution est considéré comme étant une extrémité du circuit de distribution par où s'écoule le fluide.

Selon un exemple de réalisation particulièrement avantageux, l'au moins un tube est en quartz ou en PTFE. Le quartz et le PTFE, par exemple celui commercialisé sous la marque TEFLON (marque déposée), sont en effet des matériaux ayant d'excellentes propriétés physiques concernant la transmission des rayons ultraviolets ; autrement dit, ils présentent un haut taux de transmission d'ultraviolets et permettent de minimiser les pertes de puissances en joules. En outre, le quartz et le PTFE présentent une très bonne résistance mécanique à la température et limitent des retenues de calcaire.

Par exemple, le tronçon de conduit cylindrique est en quartz, ou en PTFE.

Le tube et le tronçon de conduit cylindrique sont possiblement réalisés conjointement de manière monobloc. Selon un exemple préféré, le tube et le tronçon de conduit cylindrique sont deux pièces distinctes reliées l'une à l'autre par un deuxième ensemble d'éléments de raccord ce qui permet d'adapter facilement tout tube à une source de rayonnement primaire telle la lampe. Selon une option de réalisation, le tube a un diamètre inférieur à celui du tronçon de conduit cylindrique.

Selon un autre exemple particulièrement commode, les deux cylindres coaxiaux de la lampe sont en quartz.

Optionnellement, l'au moins un tube a une surface intérieure ou extérieure recouverte d'un revêtement contenant une substance luminescente, comme par exemple une substance à base de phosphore telle que mentionnée précédemment, propre à favoriser une transmission de rayons ultraviolet C (UV-C) depuis le tronçon de conduit cylindrique jusqu'à l'au moins un point de distribution.

Selon un exemple de réalisation privilégié, le dispositif comporte au moins un cache entourant au moins en partie l'au moins un tube jusqu'à l'au moins un point de distribution.

Optionnellement, une surface interne de l'au moins un cache est revêtue d'une matière réfléchissante, comme par exemple un chromage, ou une couche d'un composé à base de chrome ou d'aluminium, propre à améliorer un rayonnement des rayons ultraviolets sur l'au moins un tube.

Selon un mode de réalisation intéressant, le dispositif comporte au moins une source de rayonnement secondaire, disposée entre l'au moins un cache et l'au moins un tube, propre à chauffer l'au moins un tube à une température au moins égale à 70° à l'au moins un point de distribution. Une telle source de rayonnement secondaire émet de préférence un rayonnement infrarouge. Elle est particulièrement utile si le tube est contraint d'être relativement long, afin de mieux assurer une décontamination au point de distribution par exemple. La source de rayonnement secondaire est donc avantageusement positionnée au plus près possible du point de distribution.

Selon un exemple de réalisation, au moins une entrée du tronçon de conduit cylindrique est formée à une première extrémité du tronçon de conduit cylindrique et au moins une sortie du tronçon de conduit cylindrique est formée à une deuxième extrémité du tronçon de conduit cylindrique, la deuxième extrémité étant opposée à la première extrémité.

Selon un autre exemple de réalisation, l'au moins une entrée et l'au moins une sortie sont formées à une même extrémité du tronçon de conduit cylindrique.

Par exemple, l'au moins un tube comporte une prolongation pénétrant dans le tronçon de conduit cylindrique jusqu'au voisinage d'une extrémité opposée à une extrémité où est disposé le tube. Ceci permet par exemple d'augmenter un temps d'exposition du fluide au rayonnement ultraviolet.

Par exemple, le tronçon de conduit cylindrique comporte une extrémité, opposée à l'extrémité dans laquelle sont formées l'au moins une entrée et l'au moins une sortie, qui est fermée par un bouchon. Le bouchon est soit une pièce rapportée soit formée unitairement avec l'enveloppe du tronçon de conduit.

Selon encore un autre exemple de réalisation, le tronçon de conduit cylindrique comporte une première entrée à une première extrémité et une première sortie à une deuxième extrémité avec un premier tube reliant la première sortie à un premier point de distribution et dont une prolongation s'étend en outre depuis la première entrée jusqu'à la première sortie à l'intérieure du tronçon de conduit cylindrique, et en ce que le tronçon de conduit cylindrique comporte une deuxième entrée à la deuxième extrémité et une deuxième sortie à la première extrémité avec un deuxième tube reliant la deuxième sortie à un deuxième point de distribution, de sorte que le dispositif est configuré pour qu'un premier écoulement traverse la première entrée, la prolongation du premier tube à l'intérieur du tronçon de conduit cylindrique, la première sortie, le premier tube et le premier point de distribution, et pour qu'un deuxième écoulement traverse la deuxième entrée, passe dans le tronçon de conduit cylindrique autour de la prolongation du premier tube, traverse la deuxième sortie, le deuxième tube et le deuxième point de distribution. Ainsi, pour un système de distribution double, il est alors possible de n'avoir qu'une seule source de rayonnement primaire, ce qui simplifie la maintenance du dispositif.

Selon un autre aspect de la présente invention, est également proposée une fontaine à eau comportant un dispositif tel que décrit précédemment avec tout ou partie des caractéristiques précitées apportant des avantages analogues, avec l'au moins un point de distribution du dispositif formant un point de distribution de la fontaine.

D'autres avantages et caractéristiques de la présente invention ressortiront mieux à la lecture détaillée qui suit, en référence aux dessins annexés, donnés à titre illustratif et nullement limitatif, parmi lesquels :
La figure 1 représente un dispositif selon un premier mode de réalisation de la présente invention configuré pour une utilisation horizontale, La figure 2 présente le dispositif de la figure 2 en éclaté,
La figure 3 présente le dispositif des figures 1 et 2 en coupe partielle vu en perspective,
La figure 4 est un agrandissement de la zone IV de la figure 3,
La figure 5 est une vue schématique en coupe de la zone IV,
La figure 6 est une vue schématique en coupe du dispositif des figures 1 à 5 comportant en outre un système secondaire de rayonnement,
La figure 7 est une vue schématique en coupe d'un dispositif selon une première variante du premier mode de réalisation de la présente invention configuré pour une utilisation horizontale,
La figure 8 est une vue schématique en coupe d'un dispositif selon une deuxième variante du premier mode de réalisation de la présente invention configuré pour une utilisation horizontale,
La figure 9 représente un dispositif selon un deuxième mode de réalisation de la présente invention configuré pour une utilisation verticale avec flux descendant,
La figure 10 présente en perspective un intérieur du dispositif de la figure 9,
La figure 11 est une vue schématique en coupe du dispositif des figures 9 et 10,
La figure 12 est une vue schématique en coupe d'un dispositif selon une première variante du deuxième mode de réalisation de la présente invention configuré pour une utilisation verticale avec flux ascendant,
La figure 13, composée des figures 13a et 13b, présente une fontaine à eau, raccordée à un réseau d'eau courante et posée sur un support, comportant un dispositif selon un mode de réalisation de la présente invention positionné horizontalement (figure 13a) ou verticalement (figure 13b), et
La figure 14 présente une colonne de distribution d'eau réfrigérée comportant un dispositif selon un mode de réalisation de la présente invention.

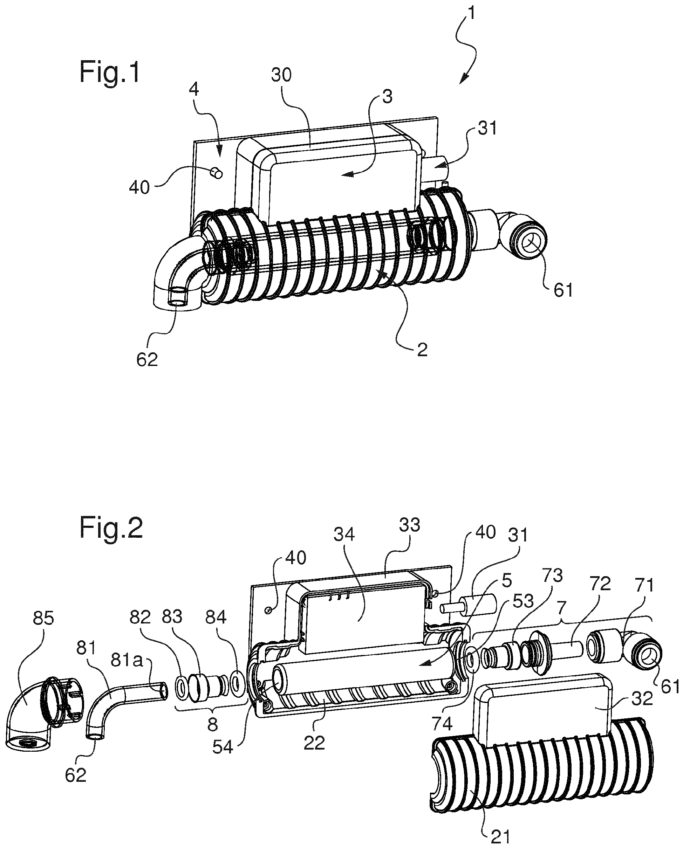

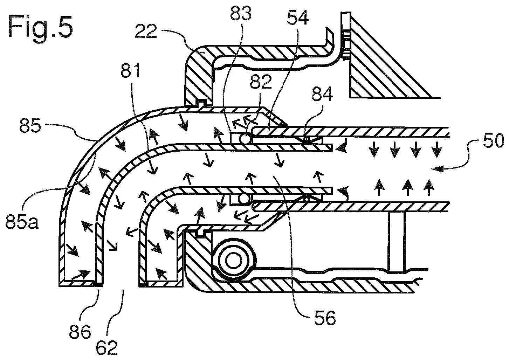

Comme l'illustrent principalement les figures 1 à 5, un dispositif 1 selon un mode de réalisation de la présente invention est un système de désinfection d'un liquide s'écoulant à travers lui. Pour cela, il comprend une entrée 61 par laquelle arrive du liquide à décontaminer, et un point de distribution 62, formant une sortie, par où s'écoule le liquide qui a été décontaminé. Un tel dispositif 1 permet par exemple de protéger de l'eau potable jusqu'au point de distribution dans un système de distribution d'eau équipé d'un stérilisateur.

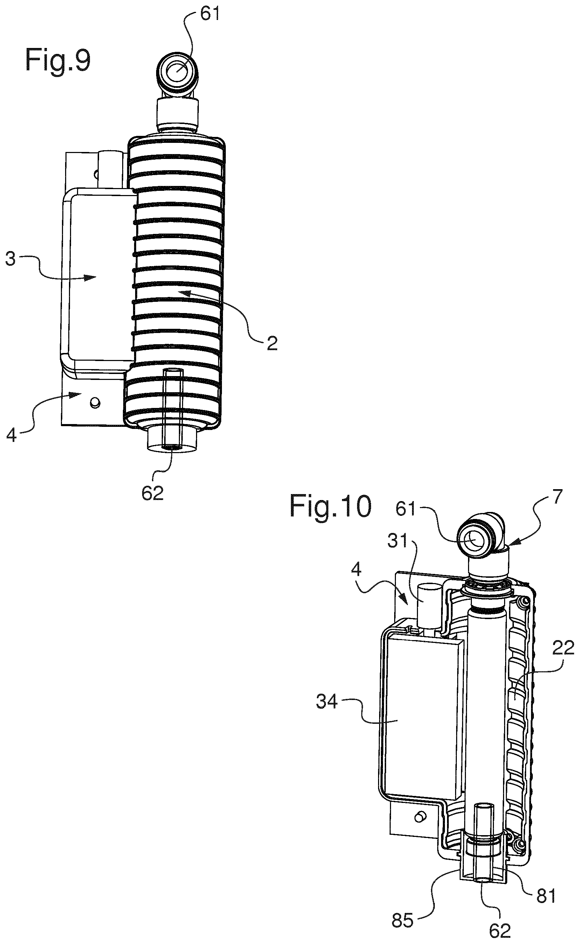

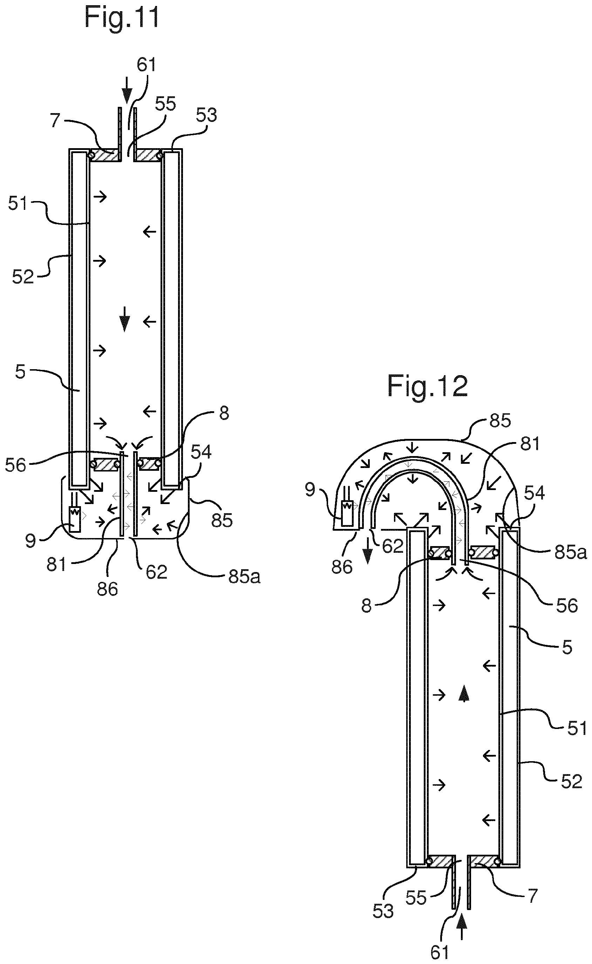

Le dispositif 1 peut être utilisé dans plusieurs positions afin de répondre aux différentes contraintes d'intégrations. Il peut être utilisé horizontalement (comme illustré sur les figures 1 à 8, 13a et 14) ou verticalement (comme illustré sur les figures 9 à 12 et 13b, avec flux descendant comme sur la figure 9 à 11 et 13b ou ascendant comme sur la figure 12).

Autrement dit, le dispositif 1 de désinfection d'un liquide par rayonnement ultraviolet comporte ici :
- au moins un conduit,
- au moins une entrée d'écoulement de liquide 61,
- au moins un point de distribution 62 formant une sortie de l'écoulement de liquide, et
- un système de rayonnement 5 comportant :
   - au moins une source de rayonnement disposée en périphérie d'un tronçon de conduit cylindrique 50,
   - le tronçon de conduit cylindrique 50 formant une partie du conduit du dispositif 1 configuré pour que du liquide s'y écoule depuis au moins une entrée 55 du système de rayonnement 5 reliée à l'au moins une entrée 61 du dispositif 1 vers au moins une sortie 56 du système de rayonnement 5 reliée à l'au moins un point de distribution 62 du dispositif 1, le tronçon de conduit 50 comportant une enveloppe 51 en un matériau configuré pour au moins transmettre une partie de rayonnement vers un intérieur du tronçon de conduit 50 destiné à recevoir l'écoulement de liquide afin de décontaminer le liquide,
      l'au moins une source de rayonnement et le tronçon de conduit 50 étant par exemple configurés pour transmettre un rayonnement tout autour de l'écoulement de liquide, et
- au moins un tube 81 comportant une enveloppe s'étendant au moins depuis l'au moins une sortie 56 du système de rayonnement 5 jusqu'à l'au moins un point de distribution 62 du dispositif 1, le tube 81 étant réalisé en un matériau configuré pour diffuser une partie de rayonnement dans une épaisseur de son enveloppe et transmettre une partie de rayonnement vers un intérieur du tube 81 destiné à recevoir l'écoulement de liquide afin de décontaminer le liquide jusqu'au point de distribution 62.

Le dispositif 1 comporte par exemple ici un carter 2 dans lequel est disposé le tronçon de tube cylindrique 50, et plus généralement ici le système de rayonnement 5. Le carter 2 est par exemple une pièce en deux parties 21, 22. Une unité 3 inclut l'ensemble des éléments électroniques 34 renfermés dans un boitier 30 qui sont nécessaires au fonctionnement du dispositif 1 dont, par exemple un ballast pour la stabilisation du courant électrique dans les circuits. L'unité 3 est alimentée par un connecteur d'alimentation 31. Elle surmonte ici le carter 2, ce qui a notamment pour avantage qu'en cas de fuite, du liquide n'atteigne pas les éléments électroniques 34. Comme le montre la figure 2, le boitier 30 est ici réalisé en deux parties 32, 33. Dans l'exemple de la figure 2, chacune des deux parties 32, 33 du boitier 30 est reliée à une des parties 32, 33 du boitier 30. Par exemple, la partie 32 du boitier et la partie 21 du carter sont réalisées ensemble et forment un capot possiblement amovible du dispositif 1, et la partie 33 du boitier et la partie 22 du carter sont réalisées ensemble.

L'unité 3 et le carter 2 sont ici fixés sur une plaque 4 servant de dissipateur de chaleur. A cet effet, celle-ci est par exemple réalisée en aluminium. La plaque 4 comprend optionnellement au moins un trou 40 permettant de faciliter sa fixation à un appareil de distribution d'eau par exemple ou à un support, comme par exemple un mur.

Dans le présent exemple de réalisation, le système de rayonnement 5 comporte, en tant que source de rayonnement ultraviolet primaire, une lampe, configurée pour générer un rayonnement ultraviolet, qui comporte deux cylindres coaxiaux 51, 52, dont un cylindre externe 52 et un cylindre interne 51 formant le tronçon de conduit cylindrique 50 délimitant une partie du conduit du dispositif 1 et configuré pour que le liquide s'y écoule depuis au moins une entrée 55 du tronçon de conduit cylindrique 50 reliée à l'au moins une entrée 61 du dispositif 1 vers au moins une sortie 56 du tronçon de conduit cylindrique 50 reliée à l'au moins un point de distribution 62 du dispositif 1 de sorte à être irradié dans son centre pour détruire virus et bactéries. Les cylindres coaxiaux 51, 52 sont en un matériau diélectrique propre à transmettre des rayons ultraviolets et sont reliés par leurs extrémités de sorte à définir entre eux une chambre hermétique.

Une telle lampe à cylindre coaxiaux permet ainsi un rayonnement réparti tout autour de l'écoulement et sur toute la longueur du tronçon de conduit cylindrique 50. La chambre hermétique est remplie ici d'un gaz de xénon pour générer le rayonnement ultraviolet, ce qui est plus écologique qu'une lampe classique. On entend par gaz de xénon un gaz comprenant au moins du xénon, et au moins une partie de paroi interne de la chambre est revêtue d'un revêtement contenant du phosphore propre à émettre des rayons UV-C. La lampe comportant en outre une première électrode positionnée sur une paroi externe et une seconde électrode, la première et la seconde électrode étant reliées à une source de courant alternatif. La première électrode est par exemple un grillage entourant la paroi externe de la lampe. La deuxième électrode a par exemple une forme de sonde, introduite au sein du cylindre interne. La première et la deuxième électrode permette ainsi à la lampe de fonctionner. Accessoirement, la lampe comprend en outre une troisième électrode, qui peut avoir une forme de sonde, qui sert à contrôler qu'un fluide est présent, c'est-à-dire qu'il y a du fluide dans le tronçon de conduit cylindrique 50, pour autoriser un fonctionnement de la lampe. Une telle lampe présente l'avantage de s'allumer très rapidement, c'est-à-dire en quelques millisecondes, et est dès lors opérationnelle. Il est ainsi possible d'allumer la lampe seulement lorsque cela est nécessaire, c'est-à-dire au moment de distribuer du liquide par exemple, et éviter ainsi qu'elle ne reste allumée en permanence, ce qui permet une économie d'énergie. Une telle lampe permet en outre de ne pas chauffer ni le tronçon de tube cylindrique ni l'écoulement, et elle a une durée de vie très longue, de l'ordre d'environ vingt mille heure de fonctionnement continu.

De manière générale, la source de rayonnement primaire émet un rayonnement UV suffisamment puissant, pour éliminer les germes et virus présents dans le liquide et dans le point de distribution 62, de manière répartie autour de l'écoulement.

Pour favoriser une répartition du rayonnement tout autour de l'écoulement, le tronçon de conduit est de préférence en quartz afin de diffuser le rayonnement dans son épaisseur et le transmettre à l'écoulement d'une manière la plus homogène possible. En effet, le quartz est un matériau permettant de bien transmettre un rayonnement ultraviolet, c'est-à-dire qu'il permet un minimum de pertes ou un meilleur taux de transmission du rayonnement et moins de pertes de puissance en joules. Un autre matériau envisageable à la place du quartz serait par exemple du polytétrafluoroéthylène (PTFE) connu notamment sous la marque TEFLON (marque déposée), ou encore tout matériau permettant de transmettre les rayons ultraviolets.

En effet, lorsqu'un rayonnement rencontre un élément en couche mince en un matériau au moins partiellement transparent aux rayons du rayonnement, une partie du rayonnement est réfléchie, une partie du rayonnement est diffusée dans une épaisseur de l'élément et une partie du rayonnement est transmise de l'autre côté de la couche par rapport à la provenance du rayonnement, c'est-à-dire qu'une partie du rayonnement traverse l'élément. Autrement dit, ici, le tronçon de conduit cylindrique 50 comporte au moins une enveloppe 51 dans un matériau tel que des rayons de la source de rayonnement s'y diffusent et soient transmis vers l'intérieur du tronçon de conduit cylindrique 50. L'écoulement de liquide à l'intérieur du tronçon de conduit cylindrique 50 reçoit ainsi des rayons émis tout autour de l'écoulement, au moins grâce à la diffusion du rayonnement dans une épaisseur de l'enveloppe 51 du tronçon de conduit cylindrique 50.

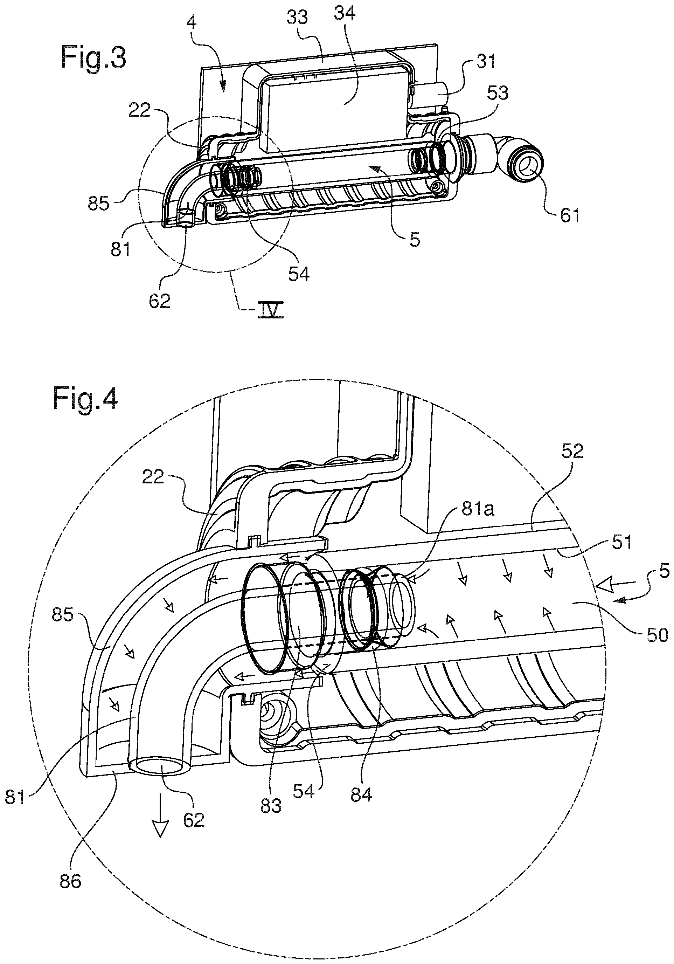

Comme le montrent les figures 2 et 3, le tronçon de conduit cylindrique 50, disposé dans le carter 2, est relié par une extrémité 53, appelée ici première extrémité 53 par commodité, à l'entrée 61 du dispositif et par une extrémité 54, appelée ici deuxième extrémité 54 par commodité, au point de distribution 62. Ainsi, le liquide entre dans le tronçon de conduit cylindrique 50 par la première extrémité 53 du tronçon de conduit cylindrique 50 et en sort par la deuxième extrémité 54. Autrement dit, une entrée 55 du système de rayonnement 5 est formée à la première extrémité 53 du tronçon de conduit cylindrique 50 et une sortie 56 du système de rayonnement 5 est formée à la deuxième extrémité 54.

Du côté de la première extrémité 53, un premier ensemble d'éléments de raccord 7, globalement tubulaires, permet de relier le dispositif 1 à une conduite de liquide d'un appareil de distribution, par exemple de façon étanche. Le premier ensemble d'éléments de raccord 7 comprend principalement, montés en série, un renvoi 71 qui est ici coudé mais qui pourrait être droit, un embout d'entrée de liquide 72, un embout d'adaptation 73 pour attacher l'embout d'entrée 72 au système de rayonnement 5, et un joint torique 74 permettant de mieux favoriser une étanchéité du montage du premier ensemble d'éléments de raccord 7. Le premier ensemble d'éléments de raccord permet de rendre le dispositif facilement amovible pour le monter et démonter d'un système de distribution de manière simple et rapide. En outre, dans le présent exemple de réalisation, l'embout d'adaptation 73 est métallique et sert ainsi d'électrode. L'embout d'adaptation 73 constitue par exemple l'une de la deuxième ou la troisième électrode telle que mentionnée précédemment.

Du côté de la deuxième extrémité 54, le dispositif 1 comporte principalement un tube 81 dont un bout 81a est relié au tronçon de conduit cylindrique 50, et en particulier à la sortie 56 du tronçon de conduit cylindrique 50, et l'autre bout forme le point de distribution 62 du dispositif 1. Pour relier le tube 81 au tronçon de conduit cylindrique 50, le dispositif 1 comprend un deuxième ensemble d'éléments de raccord 8, globalement tubulaires également, qui comprend un premier joint torique 82 destiné à être monté autour du tube 81, un embout d'adaptation 83 formé en deux parties dont une première partie est destinée à recevoir intérieurement le premier joint torique 82 et dont une deuxième partie est ici moins large que la première, et un deuxième joint torique 84 destiné à être monté autour de la deuxième partie de l'embout d'adaptation 83. En particulier, la deuxième partie de l'embout d'adaptation 83 a un diamètre moindre que la première partie. La deuxième partie de l'embout d'adaptation avec le deuxième joint torique 84 sont ainsi insérés dans le tronçon de conduit cylindrique 50 du système de rayonnement 5 tandis que la première partie de l'embout d'adaptation 83 est en butée contre un bord de la deuxième extrémité 54 de l'enveloppe 51 du tronçon de conduit cylindrique 50. En outre, dans le présent exemple de réalisation, l'embout d'adaptation 83 est métallique et sert ainsi d'électrode. L'embout d'adaptation 83 constitue par exemple l'autre de la deuxième ou la troisième électrode telle que mentionnée précédemment.

Comme le montre plus précisément la figure 4 (sur laquelle le premier joint torique 82 n'est pas représenté pour simplifier l'illustration), le bout 81a du tube 81 dépasse au-delà du deuxième ensemble d'éléments de raccord 8, vers l'intérieur système de rayonnement 5. Ceci permet d'irradier davantage le tube 81 pour améliorer la transmission du rayonnement ultraviolet jusqu'au point de distribution 62.

Le tube 81 a ici une section circulaire globalement constante. Il est en outre coudé de sorte à pointer vers le bas lorsque le dispositif 1 tel que représenté sur les figures 1 à 4 est disposé horizontalement. Il est réalisé dans un matériau transmissif des rayons UV, diélectrique, comme par exemple en quartz.

Par commodité, le tube 81 présente une section plus petite que celle du tronçon de conduit cylindrique 50. Ceci permet ainsi de maintenir le fluide dans le tronçon de conduit cylindrique 50 et de réguler l'écoulement de manière à exposer régulièrement le fluide aux radiations UV-C.

De plus, le dispositif comprend un cache réflecteur 85 qui entoure au moins en partie le tube 81. Le cache réflecteur 85 est maintenu par le carter 2. De manière générale, le carter 2 et le cache réflecteur 85 sont configurés pour enfermer l'ensemble du système de rayonnement 5 et le tube 81. Le cache réflecteur 85 a une section telle qu'un espace de forme sensiblement constante est maintenu autour du tube 81.

En outre, le cache réflecteur 85 comporte une paroi de fond 86 qui vient fermer l'espace autour du tube 81 du côté du point de distribution 62. En particulier, la paroi de fond 86 est percée d'un trou dont un rebord vient en appui sur l'extrémité du tube 81 formant le point de distribution 62.

Ainsi, le cache 85 permet de protéger le point de distribution 62 de tout contact avec un objet externe (par exemple un utilisateur ou un récipient comme un gobelet notamment) et limite ainsi les risques de contaminations bactériennes.

Ici, le tronçon de conduit cylindrique 50 pénètre au moins en partie dans le cache réflecteur 85. Ainsi, en fonctionnement, le rayonnement ultraviolet émis par le système de rayonnement 5 est transmis dans la paroi du tube 81 jusqu'au point de distribution 62 et pénètre aussi dans l'espace entre le tube 81 et le cache réflecteur 85. Par conséquent, il est préférable que le cache réflecteur 85 et l'embout d'adaptation 83 définissent entre eux un espace afin de pouvoir laisser passer le rayonnement.

Dans le présent exemple de réalisation, le cache réflecteur 85 comporte une surface interne 85a qui est revêtue d'une matière réfléchissante améliorant la radiation aux ultraviolets sur le tube 81, ce qui minimise les pertes potentielles. Ainsi, le rayonnement transmis dans la paroi du tube 81 ainsi que celui pénétrant dans l'espace entre le tube 81 et le cache réflecteur 85 viennent se projeter par réflexion dans le cache 85 sur sa surface interne 85a.

Ainsi, le tronçon de conduit cylindrique 50 vient, par sa partie pénétrant dans le cache réflecteur 85, augmenter l'intensité de radiation grâce à son rayonnement direct et par réflexion sur la surface interne 85a dans le cache réflecteur 85 pour irradier le tube 81, ce qui permet une stérilisation du tube 81 jusqu'au point de distribution 62 et ainsi une décontamination du liquide qui s'y écoule.

Le cache réflecteur 85 permet aussi de renforcer la sécurité des utilisateurs en les protégeant d'un rayonnement ultraviolet direct provenant du système de rayonnement 5.

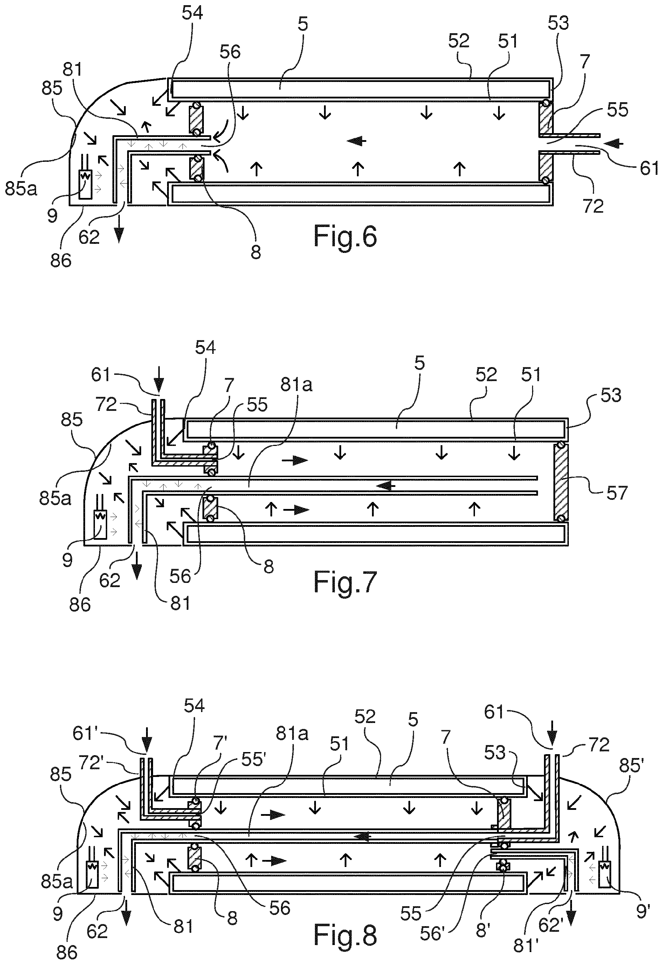

La figure 6 représente schématiquement de manière simplifiée un dispositif 1 selon un mode de réalisation présenté sur les figures 1 à 5. Ainsi, par exemple, le tube 81 est ici représenté avec un coude à angle droit, mais celui-ci serait bien entendu de préférence arrondi pour améliorer l'écoulement en minimisant les turbulences.

Sur cette figure, afin de renforcer la sécurité sanitaire et d'optimiser la durée de vie du système de rayonnement 5, une source de rayonnement secondaire 9, qui est ici une mini-lampe, est placé dans le cache réflecteur 85. La mini-lampe 9 est par exemple une lampe à rayonnement infrarouge dont le but est de chauffer, de préférence à une température au moins égale à 70°C, le tube 81 près du point de distribution 62 pour également le stériliser de façon programmé dans le temps. Une telle source de rayonnement secondaire 9 est particulièrement utile si le tube 81 est contraint d'être relativement long, afin de mieux assurer une décontamination au point de distribution 62. La source de rayonnement secondaire 9 est donc positionnée de préférence au plus près possible du point de distribution 62.

Une source de rayonnement secondaire 9 située dans le cache réflecteur 85 permet d'assurer une sécurité supplémentaire en s'allumant périodiquement par exemple, ce qui permet également d'optimiser la durée de vie du système principal.

Du point de vue du fonctionnement, dans un système UV classique une lampe à UV traditionnelle doit rester allumée en permanence ou avoir le temps d'atteindre sa température d'utilisation optimale pour délivrer sa pleine puissance. Ceci peut induire des difficultés dans le cadre d'appareils destinés à délivrer de l'eau réfrigérée par exemple, à cause d'un réchauffement engendré par la lampe, notamment si celle-ci est positionnée prêt du point de distribution. Un dispositif 1 selon l'invention avec une lampe à deux cylindres coaxiaux permet d'atteindre la pleine puissance de la lampe en quelques millisecondes et donc de s'affranchir de cette problématique de réchauffement.

Pour être adapté à une utilisation pour la stérilisation d'eau réfrigérée, le dispositif 1 fonctionne par exemple de la manière suivante :
- Mis en route du dispositif 1par un utilisateur via une commande de distribution à T0 ;
- Allumage de la lampe à pleine puissance à TO+10 ms ;
- Ouverture d'une vanne de distribution (non représentée, celle-ci se situerait en amont du point de l'entrée 61) à T0+500 ms, ce temps pouvant être étendu à quelques secondes, le but de ce décalage étant de renforcer la stérilisation du point de distribution 62 avant de distribuer l'eau ;
- A l'arrêt de la distribution par l'utilisateur par arrêt de la commande de distribution à T1, fermeture de la vanne distribution T1+1 ms ;
- Extinction de la lampe à T1+500 ms, ce temps pouvant être étendu à quelques secondes, le but de ce décalage étant de renforcer la stérilisation du point de distribution après utilisation.

Le dispositif 1 est par exemple optionnellement équipé d'un système permettant d'indiquer si le système de rayonnement 5 est hors service et la gestion électronique bloque la distribution par des vannes à réception de cette information afin de ne pas distribuer un liquide qui n'aurait pas été stérilisé par le dispositif.

Par exemple, un état de la durée de vie de la lampe peut être indiqué par une interface utilisateur et prendre la forme d'une LED lumineuse qui indique la fin de durée de vie ou d'un écran LCD indiquant les litres restants.

La figure 7 présente une première variante de réalisation d'un dispositif 1. Celle-ci diffère en ce que le dispositif 1 de la figure 7 est configuré pour une utilisation horizontale dans laquelle l'entrée 61 et le point de distribution 62 sont placés d'un même côté du système de rayonnement 5. Autrement dit, l'entrée 55 et la sortie 56 du système de rayonnement 5 sont formés à une même extrémité du tronçon de conduit cylindrique 50. Pour améliorer le temps d'irradiation du liquide, un double passage dans le système de rayonnement 5 est créé grâce à une prolongation 81a du tube 81 présente dans le tronçon de conduit cylindrique 50. Une telle prolongation 81a est par exemple un tube longiligne fixé sur le tube 81 ou est par exemple réalisé conjointement avec le tube 81 de sorte que le tube 81 et la prolongation 81a ne forment qu'une seule pièce. La prolongation 81a permet ainsi une extension du tube 81 sur, par exemple, au moins une moitié, voire au moins 80 % en longueur, du tronçon de conduit cylindrique 50 du système de rayonnement 5, de sorte à favoriser l'écoulement dans le système de rayonnement 5. Le système de rayonnement 5 est alors clos à son autre extrémité, par exemple par un bouchon 57 qui vient alors fermer l'extrémité du système de rayonnement 5 qui est opposée à celle vers laquelle sont agencés l'entrée 55 et la sortie 56, respectivement reliée à l'entrée 61 et le point de distribution 62 du dispositif 1. Un tel dispositif 1 permet ainsi de connecter le dispositif 1 au plus près d'une arrivée de liquide.

La figure 8 présente une deuxième variante de réalisation d'un dispositif 1. Celle-ci diffère en ce que le dispositif 1 de la figure 8 est configuré pour une utilisation horizontale dans laquelle le dispositif 1 comprend un système de distribution double.

En général, les systèmes de distribution double posent la problématique de la gestion de deux systèmes de rayonnement indépendants.

Un dispositif 1 selon l'invention dans ce mode de réalisation, pour simplifier la gestion, a deux points de distribution 62, 62' qui utilisent le même système de rayonnement 5 permettant de s'affranchir de la gestion de deux systèmes de rayonnement aux durées de vies différentes.

D'une part, le dispositif 1 comporte une première entrée 61 du côté d'une première extrémité 53 du tronçon de conduit cylindrique 50 du système de rayonnement 5 et une première sortie 56 formant un premier point de distribution 62 du côté d'une deuxième extrémité 54 du tronçon de conduit cylindrique 50 du système de rayonnement 5.

Le système de rayonnement 5 comporte ainsi une première entrée 55 à la première extrémité 53 et une première sortie 56 à la deuxième extrémité 54 du tronçon de conduit cylindrique 50.

Un premier tube 81 relie la première sortie 56 du système de rayonnement 5 au premier point de distribution 62 et comporte ici un prolongement 81a dans le tronçon de conduit cylindrique 50 du système de rayonnement 5 qui s'étend jusqu'à la première entrée 55 du système de rayonnement 5, elle-même raccordée à la première entrée 61 du dispositif 1 par un premier embout d'entrée 72.

D'autre part, le dispositif 1 comporte une deuxième entrée 61' du côté de la deuxième extrémité 54 et une deuxième sortie formant un deuxième point de distribution 62' du côté de la première extrémité 53 du tronçon de conduit cylindrique 50 du système de rayonnement 5.

Le système de rayonnement 5 comporte ainsi une deuxième entrée 55' à la deuxième extrémité 54 et une deuxième sortie 56' à la première extrémité 53 du tronçon de conduit cylindrique 50.

Un deuxième tube 81' relie alors la deuxième sortie 56' du système de rayonnement 5 au deuxième point de distribution 62', tandis que la deuxième entrée 55' du système de rayonnement 5 est raccordée à la deuxième entrée 61' du dispositif 1 par un deuxième embout d'entrée 72'.

Le dispositif 1 est ainsi configuré pour qu'un premier écoulement traverse la première entrée 55 du système de rayonnement 5, la prolongation 81a du premier tube 81 à l'intérieur du tronçon de conduit cylindrique 50, la première sortie 56 du système de rayonnement 5 et le premier point de distribution 62, et pour qu'un deuxième écoulement traverse la deuxième entrée 55' du système de rayonnement 5, passe dans le tronçon de conduit cylindrique 50 autour de la prolongation 81a du premier tube 81, traverse la deuxième sortie 56' du système de rayonnement 5 et le deuxième point de distribution 62'.

Les figures 9 à 12 présentent un dispositif selon un deuxième mode de réalisation et ses variantes dans un cas où celui-ci est destiné à être utilisé verticalement.

Par rapport au dispositif décrit en relation avec les figures 1 à 8, la configuration d'un dispositif 1 pour un positionnement vertical diffère principalement de par la forme du tube 81, et par conséquent du cache réflecteur 85, de sorte que le point de distribution 62 soit orienté vers le bas, c'est-à-dire pour que l'écoulement de fluide en ce point soit dirigé vers le bas, selon la gravité.

Ainsi, sur les figures 9, 10 et 11 qui présentent un dispositif 1 pour une configuration verticale avec un flux descendant, le tube 81 est droit, et le cache réflecteur 85 a globalement la même forme.

La figure 12 présentant une variante du dispositif précédent dans le cas d'un flux ascendant, le tube 81 est par conséquent recourbé en U de sorte que l'écoulement au point de distribution 62 soit orienté vers le bas, selon la gravité. Le tube 81 a par exemple une forme de crochet. Et le cache réflecteur 85 est donc adapté en conséquent.

Ainsi, de manière générale, le tube 81 peut avoir tout type de forme. Il est cependant avantageux qu'il présente une partie en sortie du système de rayonnement 5 qui soit parallèle au tronçon de conduit cylindrique 50.

Par ailleurs, une partie du tube 81 comprenant le point de distribution 62 peut également être orientée de sorte qu'un jet de liquide en sortie soit orienté si nécessaire, mais il est de préférence orienté selon la gravité pour que le jet de liquide qui en sort soit également orienté selon la gravité.

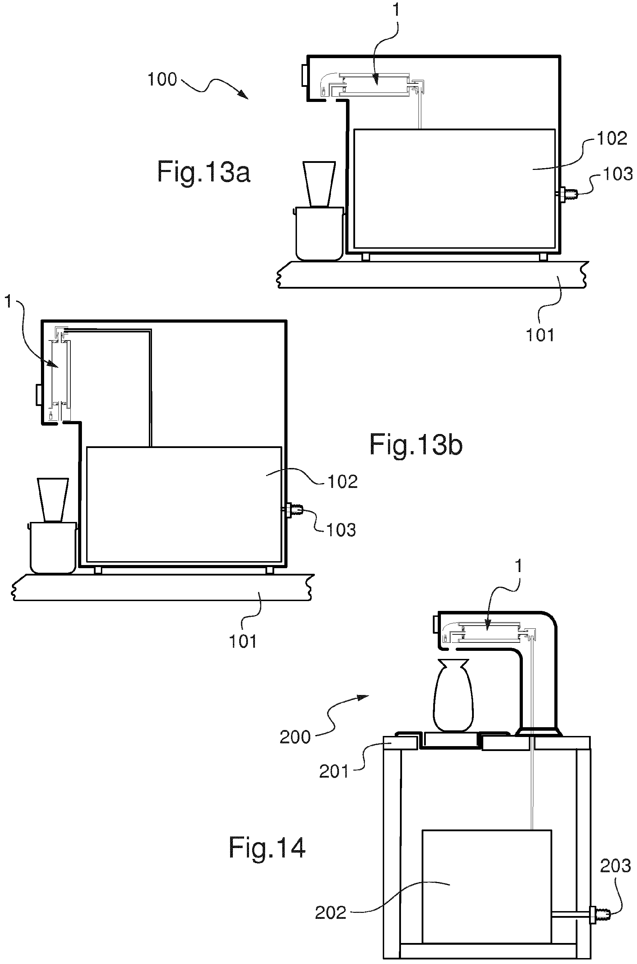

Les figures 13 et 14 présentent des exemples d'utilisation de dispositifs selon l'invention.

La figure 13a présente une fontaine 100 raccordée à un réseau d'eau courante par une entrée d'eau 103. La fontaine 100 est par exemple posée sur une paillasse ou une table ou un meuble ou tout autre support 101. Elle comporte un dispositif 1 horizontal raccordé à un groupe de réfrigération 102, disposé au sein de la fontaine 100 et lui-même raccordé à l'entrée d'eau 103.

La figure 13b présente une fontaine 100 raccordée à un réseau d'eau courante par une entrée d'eau 103 similaire à celle de la figure 13a. La différence réside dans le fait que la fontaine 100 de la figure 13b comporte un dispositif 1 vertical.

La figure 14 illustre une utilisation dans une colonne 200. La colonne 200 est également fixée sur un support 201 comme par exemple un meuble. La colonne 200 est par exemple comparable à une colonne de bière pour tirer de la bière sous pression. La colonne 200 comporte un dispositif 1 disposé horizontalement. Le dispositif 1 est raccordé à un groupe de réfrigération 202. Dans ce type d'utilisation, le groupe de réfrigération 202 est posé en bas du meuble, sous le support 201. Le groupe de réfrigération 202 est ainsi raccordé d'une part à la colonne 200 comprenant le dispositif 1 et d'autre part à une entrée d'eau 203 permettant un raccord à un réseau d'eau courante.

## Revendications

1. Dispositif de désinfection d'un liquide par rayonnement ultraviolet comportant :
- un tronçon de conduit cylindrique (50) configuré pour que du liquide s'y écoule,
- au moins une source de rayonnement ultraviolet primaire disposée en périphérie extérieure du tronçon de conduit cylindrique (50), la source de rayonnement ultraviolet primaire comportant une lampe qui comporte deux cylindres coaxiaux (51, 52), dont un cylindre externe (52) et un cylindre interne (51) formant le tronçon de conduit cylindrique (50), qui sont en un matériau diélectrique propre à transmettre des rayons ultraviolets et qui sont reliés par leurs extrémités de sorte à définir entre eux une chambre hermétique contenant un gaz, la lampe comportant en outre une première électrode et une seconde électrode, la première et la seconde électrode étant reliées à une source de courant alternatif, la lampe étant configurée pour générer un rayonnement ultraviolet entre les deux cylindres coaxiaux (51,52), le tronçon de conduit cylindrique (50) et l'au moins une source de rayonnement ultraviolet étant configurés pour que l'au moins une source de rayonnement ultraviolet irradie le tronçon de conduit cylindrique (50) et le liquide s'écoulant dans le tronçon de conduit cylindrique (50), depuis l'extérieur du tronçon de conduit cylindrique (50), et
- au moins un tube (81), formé en un matériau diélectrique propre à transmettre des rayons ultraviolets, qui prolonge le tronçon de conduit cylindrique (50) en s'étendant depuis au moins une sortie (56) du tronçon de conduit cylindrique (50) jusqu'à au moins un point de distribution (62) du dispositif (1), le tube (81) étant configuré pour guider le rayonnement ultraviolet en provenance du tronçon de conduit cylindrique (50) jusqu'à l'au moins un point de distribution (62) et irradier le liquide s'y écoulant.

2. Dispositif selon la revendication 1, **caractérisé en ce que** l'au moins un point de distribution (62) forme au moins un point d'extrémité du dispositif (1).

3. Dispositif selon l'une quelconque des revendications 1 ou 2, **caractérisé en ce que** le gaz remplissant la chambre hermétique comporte du xénon, et **en ce que** au moins une partie de paroi interne de la chambre est revêtue d'un revêtement contenant du phosphore propre à émettre des rayons UV-C.

4. Dispositif selon l'une quelconque des revendications 1 à 3, **caractérisé en ce que** l'au moins un tube (81) est en quartz ou en PTFE.

5. Dispositif selon l'une quelconque des revendications 1 à 4, **caractérisé en ce que** l'au moins un tube (81) a une surface intérieure ou extérieure recouverte d'un revêtement contenant une substance luminescente propre à favoriser une transmission de rayons ultraviolet C (UV-C) depuis le tronçon de conduit cylindrique (50) jusqu'à l'au moins un point de distribution (62).

6. Dispositif selon l'une quelconque des revendications 1 à 5, **caractérisé en ce qu'**il comporte au moins un cache (85) entourant au moins en partie l'au moins un tube (81) jusqu'à l'au moins un point de distribution (62).

7. Dispositif selon la revendication 6, **caractérisé en ce qu'**une surface interne (85a) de l'au moins un cache (85) est revêtue d'une matière réfléchissante propre à améliorer un rayonnement des rayons ultraviolets sur l'au moins un tube (81).

8. Dispositif selon l'une quelconque des revendications 6 ou 7, **caractérisé en ce qu'**il comporte au moins une source de rayonnement secondaire (9), disposée entre l'au moins un cache (85) et l'au moins un tube (81), propre à chauffer l'au moins un tube (81) à une température au moins égale à 70° à l'au moins un point de distribution (62).

9. Dispositif selon l'une quelconque des revendications 1 à 8, **caractérisé en ce qu'**au moins une entrée (55) du tronçon de conduit cylindrique (50) est formée à une première extrémité (53) du tronçon de conduit cylindrique (50) et au moins une sortie (56) du tronçon de conduit cylindrique (50) est formée à une deuxième extrémité (54) du tronçon de conduit cylindrique (50), la deuxième extrémité (54) étant opposée à la première extrémité (53).

10. Dispositif selon l'une quelconque des revendications 1 à 9, **caractérisé en ce que** l'au moins une entrée (55) et l'au moins une sortie (56) sont formées à une même extrémité (53, 54) du tronçon de conduit cylindrique (50).

11. Dispositif l'une quelconque des revendications 1 à 10, **caractérisé en ce que** l'au moins un tube (81) comporte une prolongation (81a) pénétrant dans le tronçon de conduit cylindrique (50) jusqu'au voisinage d'une extrémité opposée à une extrémité où est disposé le tube (81).

12. Dispositif selon la revendication 10, **caractérisé en ce que** le tronçon de conduit cylindrique (50) comporte une extrémité, opposée à l'extrémité dans laquelle sont formées l'au moins une entrée (55) et l'au moins une sortie (56), qui est fermée par un bouchon (57).

13. Dispositif selon l'une quelconque des revendications 1 à 11, **caractérisé en ce que** le tronçon de conduit cylindrique (50) comporte une première entrée (55) à une première extrémité (53) et une première sortie (56) à une deuxième extrémité (54) avec un premier tube (81) reliant la première sortie (56) à un premier point de distribution (62) et dont une prolongation (81a) s'étend en outre depuis la première entrée (55) jusqu'à la première sortie (56) à l'intérieure du tronçon de conduit cylindrique (50), et **en ce que** le tronçon de conduit cylindrique (50) comporte une deuxième entrée (55') à la deuxième extrémité (54) et une deuxième sortie (56') à la première extrémité (53) avec un deuxième tube (81') reliant la deuxième sortie (56') à un deuxième point de distribution (62'), de sorte que le dispositif (1) est configuré pour qu'un premier écoulement traverse la première entrée (55), la prolongation (81a) du premier tube (81) à l'intérieur du tronçon de conduit cylindrique (50), la première sortie (56), le premier tube (81) et le premier point de distribution (62), et pour qu'un deuxième écoulement traverse la deuxième entrée (55'), passe dans le tronçon de conduit cylindrique (50) autour de la prolongation (81a) du premier tube (81), traverse la deuxième sortie (56'), le deuxième tube (81') et le deuxième point de distribution (62').

14. Fontaine à eau comportant un dispositif de désinfection selon l'une quelconque des revendications 1 à 13 avec l'au moins un point de distribution (62) du dispositif formant un point de distribution de la fontaine.

## Patentansprüche

1. Vorrichtung zur Desinfektion einer Flüssigkeit durch ultraviolette Strahlung, enthaltend:
- einen zylindrischen Leitungsabschnitt (50), der dazu ausgelegt ist, dass darin eine Flüssigkeit strömt,
- zumindest eine primäre Ultraviolettstrahlungsquelle, die am äußeren Umfang des zylindrischen Leitungsabschnitts (50) angeordnet ist, wobei die primäre Ultraviolettstrahlungsquelle eine Lampe enthält, die zwei koaxiale Zylinder (51, 52), nämlich einen äußeren Zylinder (52) und einen den zylindrischen Leitungsabschnitt (50) bildenden inneren Zylinder (51), aufweist, die aus einem dielektrischen Material hergestellt sind, das für Ultraviolettstrahlen durchlässig ist, und die an ihren Enden so verbunden sind, dass sie zwischen sich eine hermetisch abgedichtete Kammer definieren, die ein Gas enthält, wobei die Lampe ferner eine erste Elektrode und eine zweite Elektrode enthält, wobei die erste und die zweite Elektrode mit einer Wechselstromquelle verbunden sind, wobei die Lampe dazu ausgelegt ist, eine ultraviolette Strahlung zwischen den beiden koaxialen Zylindern (51, 52) zu erzeugen,
wobei der zylindrische Leitungsabschnitt (50) und die zumindest eine Ultraviolettstrahlungsquelle so ausgelegt sind, dass die zumindest eine Ultraviolettstrahlungsquelle den zylindrischen Leitungsabschnitt (50) und die Flüssigkeit, die von außerhalb des zylindrischen Leitungsabschnitts (50) in den zylindrischen Leitungsabschnitt (50) einströmt, bestrahlt, und
- zumindest eine Röhre (81), die aus einem dielektrischen Material gebildet ist, das für Ultraviolettstrahlen durchlässig ist, und die den zylindrischen Leitungsabschnitt (50) fortsetzt, indem sie sich von zumindest einem Auslass (56) des zylindrischen Leitungsabschnitts (50) zu zumindest einer Abgabestelle (62) der Vorrichtung (1) erstreckt, wobei die Röhre (81) dazu ausgelegt ist, die ultraviolette Strahlung von dem zylindrischen Leitungsabschnitt (50) zu der zumindest einen Abgabestelle (62) zu leiten und die darin strömende Flüssigkeit zu bestrahlen.

2. Vorrichtung nach Anspruch 1, **dadurch gekennzeichnet, dass** die zumindest eine Abgabestelle (62) zumindest einen Endpunkt der Vorrichtung (1) bildet.

3. Vorrichtung nach Anspruch 1 oder 2, **dadurch gekennzeichnet, dass** das Gas, mit dem die hermetisch abgedichtete Kammer gefüllt ist, Xenon enthält, und dass zumindest ein Teil der Innenwand der Kammer mit einer phosphorhaltigen Beschichtung beschichtet ist, die dazu geeignet ist, UV-C-Strahlen zu emittieren.

4. Vorrichtung nach einem der Ansprüche 1 bis 3, **dadurch gekennzeichnet, dass** die zumindest eine Röhre (81) aus Quarz oder PTFE besteht.

5. Vorrichtung nach einem der Ansprüche 1 bis 4, **dadurch gekennzeichnet, dass** die zumindest eine Röhre (81) eine innere oder äußere Oberfläche aufweist, die mit einer Beschichtung bedeckt ist, die eine lumineszierende Substanz enthält, die dazu geeignet ist, ein Durchlassen von ultravioletten C-Strahlen (UV-C) vom zylindrischen Leitungsabschnitt (50) zu der zumindest einen Abgabestelle (62) zu begünstigen.

6. Vorrichtung nach einem der Ansprüche 1 bis 5, **dadurch gekennzeichnet, dass** sie zumindest eine Blende (85) umfasst, die zumindest teilweise die zumindest eine Röhre (81) bis zu der zumindest einen Abgabestelle (62) umgibt.

7. Vorrichtung nach Anspruch 6, **dadurch gekennzeichnet, dass** eine Innenfläche (85a) der zumindest einen Blende (85) mit einem reflektierenden Material beschichtet ist, das dazu geeignet ist, eine Strahlung der ultravioletten Strahlen auf die zumindest eine Röhre (81) zu verbessern.

8. Vorrichtung nach einem der Ansprüche 6 oder 7, **dadurch gekennzeichnet, dass** sie zumindest eine sekundäre Strahlungsquelle (9) enthält, die zwischen der zumindest einen Blende (85) und der zumindest einen Röhre (81) angeordnet ist und die dazu geeignet ist, die zumindest eine Röhre (81) auf eine Temperatur von zumindest gleich 70° an der zumindest einen Abgabestelle (62) zu erwärmen.

9. Vorrichtung nach einem der Ansprüche 1 bis 8, **dadurch gekennzeichnet, dass** zumindest ein Einlass (55) des zylindrischen Leitungsabschnitts (50) an einem ersten Ende (53) des zylindrischen Leitungsabschnitts (50) ausgebildet ist und zumindest ein Auslass (56) des zylindrischen Leitungsabschnitts (50) an einem zweiten Ende (54) des zylindrischen Leitungsabschnitts (50) ausgebildet ist, wobei das zweite Ende (54) dem ersten Ende (53) gegenüberliegt.

10. Vorrichtung nach einem der Ansprüche 1 bis 9, **dadurch gekennzeichnet, dass** der zumindest eine Einlass (55) und der zumindest eine Auslass (56) am gleichen Ende (53, 54) des zylindrischen Leitungsabschnitts (50) ausgebildet sind.

11. Vorrichtung nach einem der Ansprüche 1 bis 10, **dadurch gekennzeichnet, dass** die zumindest eine Röhre (81) einen Fortsatz (81a) aufweist, der in den zylindrischen Leitungsabschnitt (50) bis in die Nähe eines Endes eindringt, das einem Ende gegenüberliegt, an dem die Röhre (81) angeordnet ist.

12. Vorrichtung nach Anspruch 10, **dadurch gekennzeichnet, dass** der zylindrische Leitungsabschnitt (50) ein dem Ende, in dem der zumindest eine Einlass (55) und der zumindest eine Auslass (56) ausgebildet sind, gegenüberliegendes Ende aufweist, das von einem Stopfen (57) verschlossen ist.

13. Vorrichtung nach einem der Ansprüche 1 bis 11, **dadurch gekennzeichnet, dass** der zylindrische Leitungsabschnitt (50) einen ersten Einlass (55) an einem ersten Ende (53) und einen ersten Auslass (56) an einem zweiten Ende (54) mit einer ersten Röhre (81) aufweist, die den ersten Auslass (56) mit einer ersten Abgabestelle (62) verbindet und von welcher sich ferner ein Fortsatz (81a) von dem ersten Einlass (55) zu dem ersten Auslass (56) innerhalb des zylindrischen Leitungsabschnitts (50) erstreckt, und dass der zylindrische Leitungsabschnitt (50) einen zweiten Einlass (55') an dem zweiten Ende (54) und einen zweiten Auslass (56') an dem ersten Ende (53) mit einer zweiten Röhre (81') aufweist, die den zweiten Auslass (56') mit einer zweiten Abgabestelle (62') verbindet, so dass die Vorrichtung (1) so ausgelegt ist, dass eine erste Strömung durch den ersten Einlass (55), den Fortsatz (81a) der ersten Röhre (81) innerhalb des zylindrischen Leitungsabschnitts (50), den ersten Auslass (56), die erste Röhre (81) und die erste Abgabestelle (62) fließt, und dass eine zweite Strömung durch den zweiten Einlass (55') fließt, in den zylindrischen Leitungsabschnitt (50) um den Fortsatz (81a) der ersten Röhre (81) herum einströmt und durch den zweiten Auslass (56'), die zweite Röhre (81') und die zweite Abgabestelle (62') fließt.

14. Wasserspender mit einer Desinfektionsvorrichtung nach einem der Ansprüche 1 bis 13, wobei die zumindest eine Abgabestelle (62) der Vorrichtung eine Abgabestelle des Wasserspenders bildet.

## Claims

1. Device for disinfecting a liquid by ultraviolet radiation comprising:
- a length of cylindrical pipe (50) configured for liquid to flow therein,
- at least one source of primary ultraviolet radiation disposed at the outer periphery of the length of cylindrical pipe (50), the source of primary ultraviolet radiation comprising a lamp which comprises two coaxial cylinders (51, 52), of which one is an outer cylinder (52) and one an inner cylinder (51) forming the length of cylindrical pipe (50), which are of a dielectric material suitable for transmitting ultraviolet rays and which are connected by their ends so as to define between them a sealed chamber containing a gas, the lamp further comprising a first electrode and a second electrode, the first and the second electrode being connected to a source of alternating current, the lamp being configured to generate ultraviolet radiation between the two coaxial cylinders (51,52),
the length of cylindrical pipe (50) and the at least one source of ultraviolet radiation being configured such that the at least one source of ultraviolet radiation irradiates the length of cylindrical pipe (50) and the liquid flowing in the length of cylindrical pipe (50), from outside the length of cylindrical pipe (50), and
- at least one tube (81), formed from dielectric material suitable for transmitting ultraviolet rays, which extends the length of cylindrical pipe (50) in extending from at least one outlet (56) of the length of cylindrical pipe (50) to at least one dispensing point (62) of the device (1), the tube (81) being configured to guide the ultraviolet radiation coming from the length of cylindrical pipe (50) to the at least one dispensing point (62) and irradiate the liquid flowing therein.

2. Device according to claim 1, **characterized in that** the at least one dispensing point (62) forms at least one end point of the device (1).

3. Device according to any one of claims 1 or 2, **characterized in that** the gas filling the sealed chamber comprises xenon, and **in that** at least part of the inside wall of the chamber is coated with a coating containing phosphorus suitable for emitting UV-C rays.

4. Device according to any one of claims 1 to 3, **characterized in that** the at least one tube (81) is of quartz or of PTFE.

5. Device according to any one of claims 1 to 4, **characterized in that** the at least one (81) has an inside or outside surface covered with a coating containing a luminescent material suitable for promoting transmission of C ultraviolet (UV-C) rays from the length of cylindrical pipe (50) to the at least one dispensing point (62).

6. Device according to any one of claims 1 to 5, **characterized in that** it comprises at least one cover (85) at least partly surrounding the at least one tube (81) as far as the at least one dispensing point (62).

7. Device according to claim 6, **characterized in that** an inside surface (85a) of the at least one cover (85) is covered with a reflective material suitable for improving radiation of the ultraviolet radiation rays onto the at least one tube (81).

8. Device according to any one of claims 6 or 7, **characterized in that** it comprises at least one source of secondary radiation (9), disposed between the at least one cover (85) and the at least one tube (81), suitable for heating the at least one tube (81) to a temperature at least equal to 70° at the at least one dispensing point (62).

9. Device according to any one of claims 1 to 8, **characterized in that** at least one entry (55) of the length of cylindrical pipe (50) is formed at a first end (53) of the length of cylindrical pipe (50) and at least one exit (56) of the length of cylindrical pipe (50) is formed at a second end (54) of the length of cylindrical pipe (50), the second end (54) being opposite the first end (53).

10. Device according to any one of claims 1 to 9, **characterized in that** the at least one entry (55) and the at least one exit (56) are formed at a same end (53, 54) of the length of cylindrical pipe (50).

11. Device according to any one of claims 1 to 10, **characterized in that** the at least one tube (81) comprises an extension (81a) entering into the length of cylindrical pipe (50) as far as the vicinity of an opposite end from the end at which is disposed the tube (81).

12. Device according to claim 10, **characterized in that** the length of cylindrical pipe (50) comprises an end, which is an opposite end to the end in which are formed the at least one entry (55) and the at least one exit (56), which is closed by a plug (57).

13. Device according to any one of claims 1 to 11, **characterized in that** the length of cylindrical pipe (50) comprises a first entry (55) at a first end (53) and a first exit (56) at a second end (54) with a first tube (81) connecting the first exit (56) to a first dispensing point (62) and of which an extension (81a) furthermore extends from the first entry (55) to the first exit (56) inside the length of cylindrical pipe (50), and **in that** the length of cylindrical pipe (50) comprises a second entry (55') at the second end (54) and a second exit (56') at the first end (53) with a second tube (81') connecting the second exit (56') to a second dispensing point (62'), such that the device (1) is configured in order for a first flow to pass through the first entry (55), the extension (81a) of the first tube (81) inside the length of cylindrical pipe (50), the first exit (56), the first tube (81) and the first dispensing point (62), and in order for a second flow to pass through the second entry (55'), pass within the length of cylindrical pipe (50) around the extension (81a) of the first tube (81), pass through the second exit (56'), the second tube (81') and the second dispensing point (62').

14. Water dispenser comprising a disinfection device according to any one of claims 1 to 13 with the at least one dispensing point (62) of the device forming a dispensing point of the dispenser.
